# EUROPEAN PATENT SPECIFICATION

(11) **EP 4 471 011 B1**
(45) Date of publication and mention of the grant of the patent: **14.01.2026**
(21) Application number: 23305863.5
(22) Date of filing: 01.06.2023
(51) Int. Cl.: C07D 207/323, C07C 15/00, C07C 17/00, C07C 41/18, C07C 67/28, C07C 67/30, C07D 209/08, C07D 277/64, C07D 307/79, C07D 333/54, C07B 31/00, C07C 1/32, C07C 67/317, C07C 17/361

(54) **PROCESS FOR THE SELECTIVE CLEAVAGE OF C-SCF3 BONDS AND ANALOGUES**
VERFAHREN ZUR SELEKTIVEN SPALTUNG VON C-SCF3-BINDUNGEN UND ANALOGA
PROCÉDÉ DE CLIVAGE SÉLECTIF DE LIAISONS C-SCF3 ET ANALOGUES

(43) Date of publication of application: 04.12.2024
(73) Proprietor: Centre National de la Recherche Scientifique, 75016 Paris (FR); Institut National des Sciences Appliquées de Rouen Normandie, 76801 Saint-Etienne-du-Rouvray, Cedex (FR); Université de Rouen Normandie, 76130 Mont-Saint-Aignan (FR)
(72) Inventor: BESSET, Tatiana, 76230 ISNEAUVILLE (FR); CASTANHEIRO, Matias, 76770 MALAUNAY (FR); NOBILE, Enzo, 91300 MASSY (FR); ARRIBAT, Mathieu, 27460 Alizay (FR)
(74) Representative: Santarelli

(56) References cited:
- WO-A1-2008/040057
- WO-A1-2021/018572
- SASAKI HIROTAKA ET AL: "Nucleophilic addition reactions of benzoxanthone derivatives with collapse of aromaticity", TETRAHEDRON, vol. 76, no. 39, 1 September 2020 (2020-09-01), AMSTERDAM, NL, pages 131472, XP093101582, ISSN: 0040-4020, DOI: 10.1016/j.tet.2020.131472
- MARTIN R BRYCE ET AL: "Formation and rearrangement of adducts from benzyne and substituted 2,1,3-benzoselenadiazoles", JOURNAL OF THE CHEMICAL SOCIETY, PERKIN TRANSACTIONS 1, ROYAL SOCIETY OF CHEMISTRY, CAMBRIDGE, UK, 1 January 1981 (1981-01-01), pages 607 - 613, XP008152039, ISSN: 0300-922X, DOI: 10.1039/P19810000607
- ZHENGKUN Y ET AL: "Mild and Efficient Desulfurization of Alkyl Sulfides with Sodium", TETRAHEDRON LETTERS, ELSEVIER, AMSTERDAM , NL, vol. 39, no. 18, 30 April 1998 (1998-04-30), pages 2671 - 2674, XP004113316, ISSN: 0040-4039, DOI: 10.1016/S0040-4039(98)00368-2
- BARBERO NEKANE ET AL: "Ligand-Free Ni-Catalyzed Reductive Cleavage of Inert Carbon-Sulfur Bonds", vol. 14, no. 3, 18 January 2012 (2012-01-18), US, pages 796 - 799, XP093101949, ISSN: 1523-7060, Retrieved from the Internet <URL:https://pubs.acs.org/doi/epdf/10.1021/ol2033306> DOI: 10.1021/ol2033306

## Description

The present invention concerns a process for the selective cleavage of C-SCF₃ bonds and analogues of such bonds.

In a society concerned about the environment, earth and aquatic systems pollution and circular economy, development of new technologies to meet such objectives is nowadays a real challenge and a hot topic across the whole chemical industry.

The field of organofluorine chemistry is unavoidable and fluorinated compounds are highly represented in many fields such as materials science, the pharmaceutical and agrochemical industries. Indeed, the incorporation of a fluorine atom or a fluorinated unit can modify the physico-chemical properties of organic molecules in a significant way explaining the interest of the scientific community regarding this research area. However, the prevalence of these compounds raises the question of their fate and degradation. Recently, there has been a strong awareness from the scientific community and society regarding chlorofluorocarbons (CFCs), hydrofluorocarbons (HFCs) and per- and poly-fluoroalkyl substances (PFAs). It is now important to go even further and to look at the fate of emerging fluorinated groups that have become essential, such as derivatives containing a SCF₃ unit (eg. Toltrazuril and Fipronil).

In this context, the development of tools allowing to valorize these fluorinated derivatives, which, once used, are then considered as waste by converting them into compounds with high added value would have an undeniable ecological impact and allow for cost reduction. It is therefore essential to develop innovative tools to meet this challenge and to remove this synthetic lock.

M. R. Bryce et al., J. Chem. Soc. Perkin Trans. 1, 1981, pages 607-613 discloses reductive deselenisation of an arylselenylcyanide using Raney nickel. Barbero Nekane et al., Org. Lett., 14 (3), 2012, pages 796-799 discloses the Ni-catalysed reductive cleavage of carbon-sulfur bonds using Ni(COD)₂ and EtMe₂Si. WO 2021/018572 discloses Ni-catalysed reductive cleavage of carbon-sulfur bonds using Ni(4-CF₃stb)₃ and EtMe₂SiH.

There is currently no technology allowing to selectively cleave C(sp²)-SRf bonds on (hetero)aromatic derivatives or vinyl positions or C(sp³)-SRf bonds and any progress would have a major impact from a scientific and environmental point of view.

One aim of the present invention is thus to provide a tool for the de-fluorination of fluorinated compounds.

One aim of the present invention is to provide a process for the de-fluorination of fluorinated compounds, for example through the cleavage of C-SCF₃ bonds, or analogues of such bonds.

Thus, the present invention relates to a process for the preparation of a compound having the formula (I):

A-H

by reacting a compound having the formula (II):

   A-X
in the presence of a metallic complex comprising nickel, a ligand, a hydride, and a solvent,
wherein:
   - A is selected from the group consisting of:
      - (C₆-C₁₀)aryl groups,
         said aryl groups being optionally substituted with at least one substituent preferably selected from the group consisting of: halogen, (C₁-C₆)alkyl, (C₁-C₆)alkoxy, -CN, -CF₃, -OCF₃, -NRₐR_{b}, -SiRₐR_{b}R_{c}, -BRₐR_{b}, (C₂-C₆)alkenyl, (C₂-C₆)alkynyl, -C(=O)-NRₐR_{b}, and -C(=O)-O(C₁-C₆)alkyl;
         Rₐ and R_{b} being independently from each other H or a (C₁-C₆)alkyl group; R_{c} being a (C₁-C₆)alkyl group;
      - heteroaryl groups comprising from 5 to 10 atoms and including at least one heteroatom selected from O, N, and S,
         said heteroaryl groups being optionally substituted with at least one substituent preferably selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
      - a vinyl compound having the following formula (III): 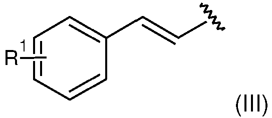 R¹ being selected from the group consisting of: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
   - X is selected from the group consisting of: YCF₃, YOCF₃, YO₂CF₃, YCF₂SO₂Ph, YOCF₂SO₂Ph, YO₂CF₂SO₂Ph, Y(O)ₙCF₂COOR, Y(O)ₙCF₂CONR₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR, Y being S or Se, Y being preferably S, n being 1 or 2, and R being (C₁-C₆)alkyl.

According to an embodiment, in formula (I) or (II) above, A is selected from the group consisting of:
- (C₆-C₁₀)aryl groups,
   said aryl groups being optionally substituted with at least one substituent selected from the group consisting of: (C₁-C₆)alkyl, (C₁-C₆)alkoxy, and -C(=O)-O(C₁-C₆)alkyl;
- heteroaryl groups comprising from 5 to 10 atoms and including at least one heteroatom selected from O, N, and S,
   said heteroaryl groups being optionally substituted with at least one substituent selected from: (C₁-C₆)alkyl, (C₁-C₆)alkoxy, and -C(=O)-O(C₁-C₆)alkyl; and
- a vinyl compound having the following formula (III): 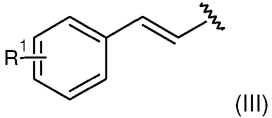 R¹ being selected from the group consisting of: (C₁-C₆)alkyl, (C₁-C₆)alkoxy, and -C(=O)-O(C₁-C₆)alkyl.

According to an embodiment, in formula (II) above, X is selected from the group consisting of: SCF₃, SOCF₃, SO₂CF₃, SCF₂SO₂Ph, SOCF₂SO₂Ph, SO₂CF₂SO₂Ph, S(O)ₙCF₂COOR, S(O)ₙCF₂CONR₂, S(O)ₙCF₂CH₂OH, S(O)ₙCN, S(O)ₙCHF₂, S(O)ₙCF₂H, S(O)ₙCF₂CₙF₂ₙ₊₁, S(O)ₙCF₂COR, and all analogues of this list in the selenium series, R and n being as defined above.

According to an embodiment, in formula (II) above, X is selected from the group consisting of: SCF₃, SOCF₃, SO₂CF₃, SCF₂SO₂Ph, SOCF₂SO₂Ph, SO₂CF₂SO₂Ph, S(O)ₙCF₂COOR, S(O)ₙCF₂CONR₂, S(O)ₙCF₂CH₂OH, S(O)ₙCN, S(O)ₙCHF₂, S(O)ₙCF₂H, S(O)ₙCF₂CₙF₂ₙ₊₁, S(O)ₙCF₂COR, and SeCF₃.

According to an embodiment, in formula (II) above, X is selected from the group consisting of: SCF₃, SOCF₃, SO₂CF₃, SCF₂SO₂Ph, SO₂CF₂SO₂Ph, and SeCF₃.

The following definitions are set forth to illustrate and define the meaning and scope of the various terms used to describe the invention herein.

The expression "Cₜ-C_{z}" means a carbon-based chain which can have from t to z carbon atoms, for example C₁-C₃ means a carbon-based chain which can have from 1 to 3 carbon atoms.

The term "alkyl group" means: a linear or branched, saturated, hydrocarbon-based aliphatic group comprising, unless otherwise mentioned, from 1 to 12 carbon atoms. By way of examples, mention may be made of methyl, ethyl, n-propyl, isopropyl, butyl, isobutyl, tert-butyl or pentyl groups.

The term "aryl group" means: a cyclic aromatic group comprising between 6 and 10 carbon atoms. By way of examples of aryl groups, mention may be made of phenyl or naphthyl groups.

The term "heteroaryl group" means: a 5- to 10-membered aromatic monocyclic or bicyclic group containing from 1 to 4 heteroatoms selected from O, S or N. By way of examples, mention may be made of imidazolyl, thiazolyl, oxazolyl, furanyl, thiophenyl, pyrazolyl, oxadiazolyl, tetrazolyl, pyridinyl, pyrazinyl, pyrimidinyl, pyridazinyl, indolyl, benzofuranyl, benzothiophenyl, benzoxazolyl, benzimidazolyl, indazolyl, benzothiazolyl, isobenzothiazolyl, benzotriazolyl, quinolinyl and isoquinolinyl groups.

By way of a heteroaryl comprising 5 to 6 atoms, including 1 to 4 nitrogen atoms, mention may in particular be made of the following representative groups: pyrrolyl, pyrazolyl, 1,2,3-triazolyl, 1,2,4-triazolyl, tetrazolyl and 1,2,3-triazinyl.

Mention may also be made, by way of heteroaryl, of thiophenyl, oxazolyl, furazanyl, 1,2,4-thiadiazolyl, naphthyridinyl, quinoxalinyl, phthalazinyl, imidazo[1,2-a]pyridine, imidazo[2,1-b]thiazolyl, cinnolinyl, benzofurazanyl, azaindolyl, benzimidazolyl, benzothiophenyl, thienopyridyl, thienopyrimidinyl, pyrrolopyridyl, imidazopyridyl, benzoazaindole, 1,2,4-triazinyl, indolizinyl, isoxazolyl, isoquinolinyl, isothiazolyl, purinyl, quinazolinyl, quinolinyl, isoquinolyl, 1,3,4-thiadiazolyl, thiazolyl, isothiazolyl, carbazolyl, and also the corresponding groups resulting from their fusion or from fusion with the phenyl nucleus.

When an alkyl group is substituted with an aryl group, the term "arylalkyl" or "aralkyl" group is used. The "arylalkyl" or "aralkyl" groups are aryl-alkyl- groups, the aryl and alkyl groups being as defined above. Among the arylalkyl groups, mention may in particular be made of the benzyl or phenethyl groups.

The term "halogen" means: a fluorine, a chlorine, a bromine or an iodine.

The term "alkoxy group" means: an -O-alkyl group where the alkyl group is as previously defined. By way of examples, mention may be made of -O-(C₁-C₄)alkyl groups, and in particular the -O-methyl group, the -O-ethyl group, as -O-C₃alkyl group, the -O-propyl group, the -O-isopropyl group, and as -O-C₄alkyl group, the -O-butyl, - O-isobutyl or -O-tert-butyl group.

The term "alkynyl" as employed herein includes unsaturated, nonaromatic, hydrocarbon groups having 2 to 6 carbons, and comprising at least one triple bond. Preferably, the alkynyl group is linear. Preferably, the alkynyl group is a -(CH₂)ₘ-C≡CH group, m being an integer comprised from 1 to 4.

The term "alkenyl" as employed herein includes unsaturated, nonaromatic, hydrocarbon groups having 2 to 6 carbons, and comprising at least one double bond. Preferably, the alkenyl group is linear. Preferably, the alkenyl group is a -(CH₂)ₘ-CH=CH₂ group, m being an integer comprised from 1 to 4.

The abovementioned "alkyl", "aryl", and "heteroaryl" groups can be substituted with one or more substituents. Among these substituents, mention may be made of the following groups: amino, hydroxyl, thiol, oxo, halogen, alkyl, alkoxy, alkylthio, alkylamino, aryloxy, arylalkoxy, cyano, trifluoromethyl, carboxy or carboxyalkyl.

The term "alkylthio" means: an -S-alkyl group, the alkyl group being as defined above.

The term "alkylamino" means: an -NH-alkyl group, the alkyl group being as defined above.

The term "aryloxy" means: an -O-aryl group, the aryl group being as defined above.

The term "arylalkoxy" means: an aryl-alkoxy- group, the aryl and alkoxy groups being as defined above.

The term "carboxyalkyl" means: an HOOC-alkyl- group, the alkyl group being as defined above. As examples of carboxyalkyl groups, mention may in particular be made of carboxymethyl or carboxyethyl.

The term "haloalkyl group" means: an alkyl group as defined above, in which one or more of the hydrogen atoms is(are) replaced with a halogen atom. By way of example, mention may be made of fluoroalkyls, in particular CF₃ or CHF₂.

The term "carboxyl" means: a COOH group.

The term "oxo" means: "=O".

In one embodiment of the process according to the invention, the hydride is a silane compound of formula R²(R³)₂SiH, R² and R³ being independently from each other selected from (C₁-C₆)alkyl, (C₁-C₆)alkoxy, and (C₆-C₁₀)aryl groups, said aryl groups being optionally substituted with at least one substituent selected from: (C₁-C₆)alkyl, (C₁-C₆)alkoxy, -CN, -OCF₃, -NRₐR_{b}, -Si(R_{c})₃, -BRₐR_{b}, (C₂-C₆)alkenyl, (C₂-C₆)alkynyl, -C(=O)-NRₐR_{b}, -C(=O)-O(C₁-C₆)alkyl, and CF₃, Rₐ, R_{b} and R_{c} being as defined above.

In one embodiment of the process according to the invention, the hydride is a silane compound of formula R²(R³)₂SiH, R² and R³ being independently from each other selected from (C₁-C₆)alkyl, (C₁-C₆)alkoxy, and (C₆-C₁₀)aryl groups, said aryl groups being optionally substituted with at least one substituent selected from: (C₁-C₆)alkyl, (C₁-C₆)alkoxy, and -C(=O)-O(C₁-C₆)alkyl, and CF₃.

In one embodiment of the process according to the invention, the hydride is selected from the group consisting of: EtMe₂SiH, Me(OMe)₂SiH, Me(OEt)₂SiH, (OEt)₃SiH, Et₃SiH, tBuMe₂SiH, Me₂PhSiH, Me₂BnSiH, MePh₂SiH, Ph₃SiH, Me₂(*p-*CF₃(C₆H₄))SiH, and Me₂(*p*-tBu(C₆H₄))SiH.

In one embodiment, the hydride is Me₂(*p*-CF₃(C₆H₄))SiH or Me₂BnSiH.

According to one embodiment, in the process according to the invention, the ligand is selected from the group consisting of: 1,5-cyclooctadiene (COD), 1,2-Bis(dicyclohexylphosphino)ethane (dcype), 1,1'-Bis(diphenylphosphino)ferrocene (dppf), 1,1'-Bis(di-*tert*-butylphosphino)ferrocene (DTBPF), 4,5-Bis(diphenyl-phosphino)-9,9-dimethylxanthene (XantPhos), and triphenylarsine (AsPh₃).

Preferably, the metallic complex is a Ni(0) complex such as bis(1,5-cyclooctadiene)nickel.

According to one embodiment, the solvent used in the process according to the invention is selected from the usual solvents used in the field. Preferably, the solvent for the process according to the invention is toluene.

According to one embodiment of the process according to the invention, the reaction is carried out a temperature from 90°C to 140°C. Preferably, the reaction may be carried out at 90°C, 120°C, and 140°C.

The present invention also relates to a process as defined above, for the preparation of a compound having the formula (I), from a compound having the formula A-SCF₃ (11-1),
wherein A is a heteroaryl group comprising from 5 to 10 atoms and including at least one heteroatom selected from O, N, and S, said heteroaryl group being optionally substituted with at least one substituent selected from: halogen, (C₁-C₆)alkyl, (C₁-C₆)alkoxy, and -C(=O)-O(C₁-C₆)alkyl.

The present invention also relates to a process as defined above, for the preparation of a compound having the formula (I-2): 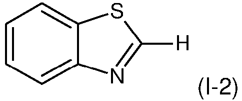 from a compound having the formula (II-2):

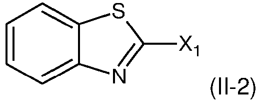

X₁ being selected from the group consisting of: SCF₃, SOCF₃, SO₂CF₃, SCF₂SO₂Ph, SOCF₂SO₂Ph, SO₂CF₂SO₂Ph, and SeCF₃.

According to one embodiment of the processes as mentioned above, the reaction is carried out in the presence of toluene with bis(1,5-cyclooctadiene)nickel and Me₂(*p*-CF₃(C₆H₄))SiH.

The present invention also relates to a process as defined above, for the preparation of a compound having the formula (I-3): 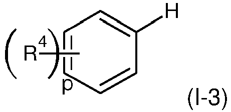 from a compound having the formula (II-3): 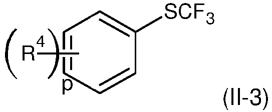
p being 0, 1, 2, or 3; and
R⁴, identical or different, being selected from the group consisting of: (C₁-C₆)alkyl, (C₁-C₆)alkoxy, and -C(=O)-O(C₁-C₆)alkyl.

The present invention also relates to a process as defined above, for the preparation of a compound having the formula (I-4): 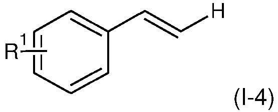 from a compound having the formula (II-4): 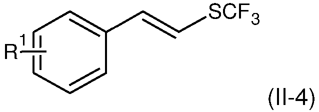 R¹ being selected from the group consisting of: halogen, (C₁-C₆)alkyl, and -O-C(=O)-(C₁-C₆)alkyl.

According to one embodiment of the above processes, the reaction is carried out in the presence of toluene, a base and molecular sieve with bis(1,5-cyclooctadiene)nickel and BnMe₂SiH.

Preferably, the base is K₂HPO₄.

### EXAMPLES

### General procedures for the reductive cleavage

**General procedure A:** an oven-dried 10 mL tube equipped with a stirring bar was charged with Ni(cod)₂ (14 mg, 0.05 mmol, 10 mol%), (hetero)aryl-SCF₃ derivative **1a-k** or **3-7** (0.5 mmol, 1 equiv.), Me₂(*p*-CF₃(C₆H₄))SiH (408 mg, 2 mmol, 4 equiv.) and toluene (2 mL) under argon. The resulting solution was stirred at 140 °C for 48 h. The mixture was allowed to cooled down to 21 °C. The solution was filtered on CHROMAFIL^{®} Xtra PTFE-45/25 and the solvent was evaporated under reduced pressure. The residue was purified by silica gel flash column chromatography by dry loading the samples and eluting with a solvent system as noted below to afford the desired products **2a-k.**

The steps, the reactants, the structures of the compounds, and the corresponding yields are shown in Scheme 1 hereafter.

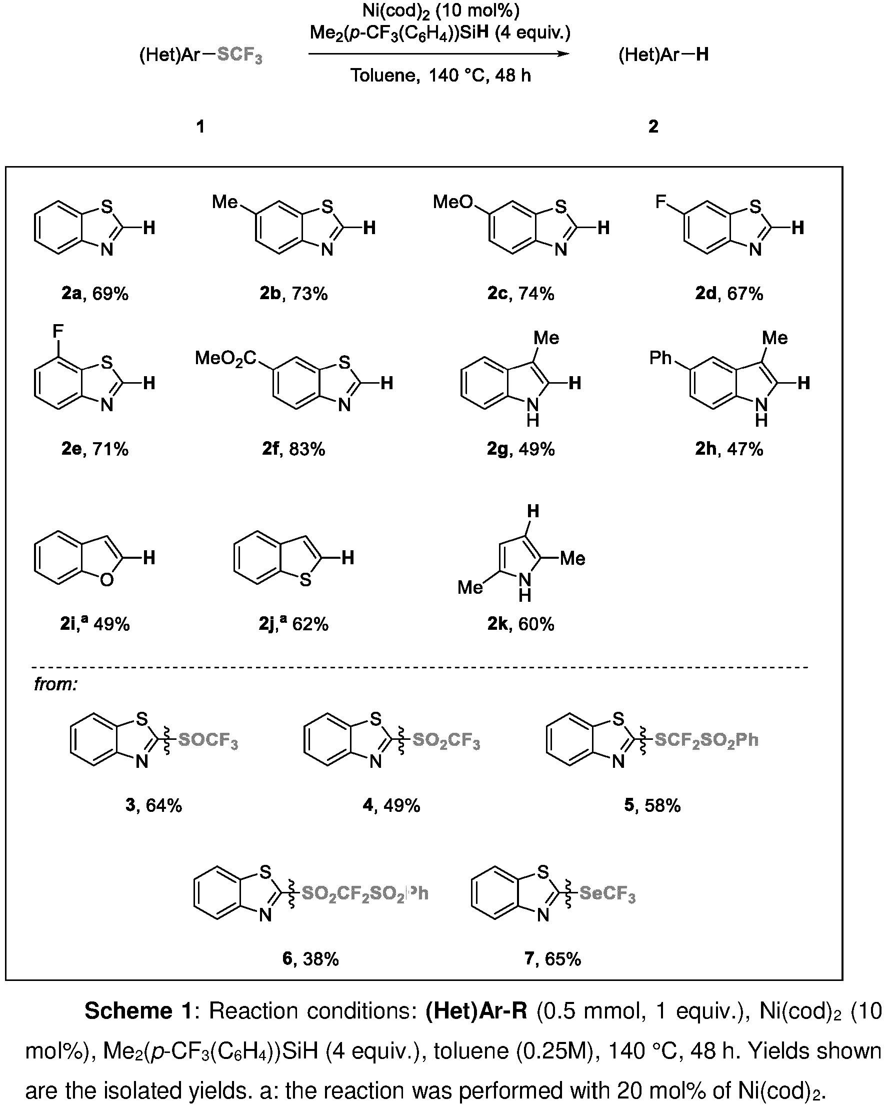

**General procedure B:** an oven-dried 10 mL tube equipped with a stirring bar was charged with Ni(cod)₂ (14 mg, 0.05 mmol, 10 mol%), aryl-SCF₃ **8** or vinyl-SCF₃ **10** derivatives (0.5 mmol, 1 equiv.), K₂HPO₄ (174 mg, 1 mmol, 2 equiv.), Me₂BnSiH (301 mg, 2 mmol, 4 equiv.), toluene (2 mL) and 4Å MS (62 mg) under argon. The resulting solution was stirred at 140°C for 48 h. The mixture was allowed to cooled down to 21°C. The solution was filtered on CHROMAFIL^{®} Xtra PTFE-45/25 and the solvent was evaporated under reduced pressure. The residue was purified by silica gel flash column chromatography by dry loading the samples and eluting with a solvent system as noted below to afford the desired products **9** or **11.**

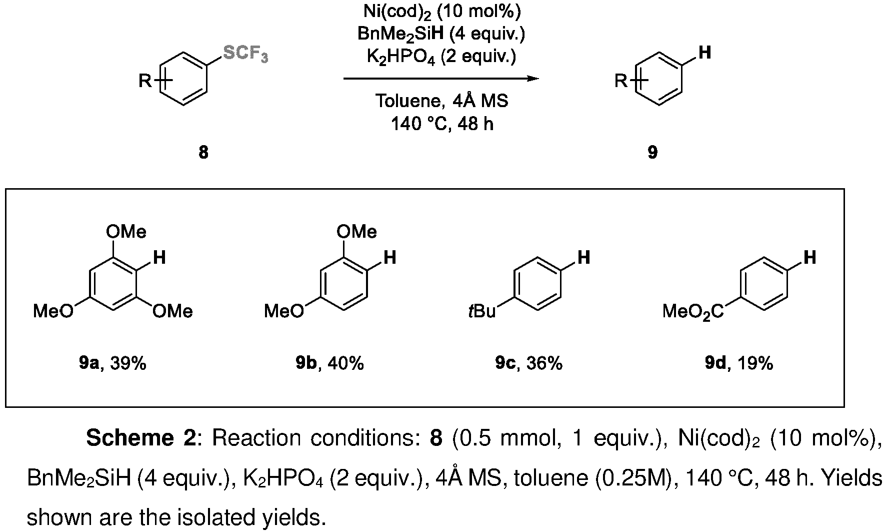

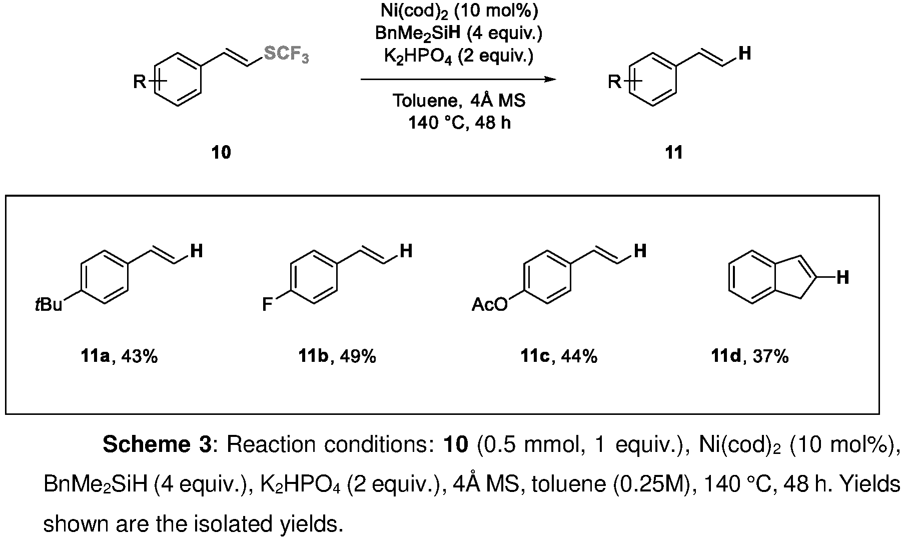

## Claims

1. A process for the preparation of a compound having the formula (I):
A-H
by reacting a compound having the formula (II):
A-X
in the presence of a metallic complex comprising nickel, a ligand, a hydride, and a solvent,
wherein:
- A is selected from the group consisting of:
. (C₆-C₁₀)aryl groups,
said aryl groups being optionally substituted with at least one substituent preferably selected from the group consisting of: halogen, (C₁-C₆)alkyl, (C₁-C₆)alkoxy, -CN, -CF₃, -OCF₃, -NRₐR_{b}, -SiRₐR_{b}R_{c}, -BRₐR_{b}, (C₂-C₆)alkenyl, (C₂-C₆)alkynyl, -C(=O)-NRₐR_{b}, and -C(=O)-O(C₁-C₆)alkyl;
Rₐ and R_{b} being independently from each other H or a (C₁-C₆)alkyl group; R_{c} being a (C₁-C₆)alkyl group;
. heteroaryl groups comprising from 5 to 10 atoms and including at least one heteroatom selected from O, N, and S,
said heteroaryl groups being optionally substituted with at least one substituent preferably selected from: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
. a vinyl compound having the following formula (III): 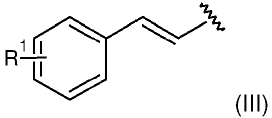 R¹ being selected from the group consisting of: halogen, (C₁-C₆)alkyl, (C₁-C₆)alkoxy, -CN, -CF₃, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, Rₐ, R_{b} and R_{c} being as defined above; and
- X is selected from the group consisting of: YCF₃, YOCF₃, YO₂CF₃, YCF₂SO₂Ph, YOCF₂SO₂Ph, YO₂CF₂SO₂Ph, Y(O)ₙCF₂COOR, Y(O)ₙCF₂CONR₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR, Y being S or Se, n being 1 or 2, and R being (C₁-C₆)alkyl.

2. The process of claim 1, wherein the hydride is a silane compound of formula R²(R³)₂SiH, R² and R³ being independently from each other selected from (C₁-C₆)alkyl, (C₁-C₆)alkoxy, and (C₆-C₁₀)aryl groups, said aryl groups being optionally substituted with at least one substituent selected from: (C₁-C₆)alkyl, (C₁-C₆)alkoxy, -CN, -OCF₃, -NRₐR_{b}, -Si(R_{c})₃, -BRₐR_{b}, (C₂-C₆)alkenyl, (C₂-C₆)alkynyl, -C(=O)-NRₐR_{b}, and -C(=O)-O(C₁-C₆)alkyl, and CF₃, Rₐ, R_{b} and R_{c} being as defined in claim 1.

3. The process of claim 1 or 2, wherein the hydride is selected from the group consisting of: EtMe₂SiH, Me(OMe)₂SiH, Me(OEt)₂SiH, (OEt)₃SiH, Et₃SiH, tBuMe₂SiH, Me₂PhSiH, Me₂BnSiH, MePh₂SiH, Ph₃SiH, Me₂(*p*-CF₃(C₆H₄))SiH, and Me₂(*p-*tBu(C₆H₄))SiH.

4. The process of any one of claims 1 to 3, wherein the hydride is Me₂(*p-*CF₃(C₆H₄))SiH or Me₂BnSiH.

5. The process of any one of claims 1 to 4, wherein the ligand is selected from the group consisting of: 1,5-cyclooctadiene (COD), 1,2-Bis(dicyclohexylphosphino)ethane (dcype), 1,1'-Bis(diphenylphosphino)ferrocene (dppf), 1,1'-Bis(di-*tert*-butylphosphino)ferrocene (DTBPF), 4,5-Bis(diphenylphosphino)-9,9-dimethylxanthene (XantPhos), and triphenylarsine (AsPh₃).

6. The process of any one of claims 1 to 5, wherein the solvent is toluene.

7. The process of any one of claims 1 to 6, wherein the reaction is carried out at a temperature from 90°C to 140°C.

8. The process of any one of claims 1 to 7, wherein the metallic complex is a Ni(0) complex such as bis(1,5-cyclooctadiene)nickel.

9. The process of any one of claims 1 to 8, for the preparation of a compound having the formula (I), from a compound having the formula A-SCF₃ (II-1),
wherein A is a heteroaryl group comprising from 5 to 10 atoms and including at least one heteroatom selected from O, N, and S, said heteroaryl group being optionally substituted with at least one substituent selected from: halogen, (C₁-C₆)alkyl, (C₁-C₆)alkoxy, and -C(=O)-O(C₁-C₆)alkyl.

10. The process of any one of claims 1 to 8, for the preparation of a compound having the formula (I-2): 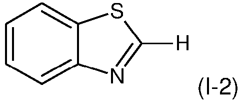 from a compound having the formula (II-2): 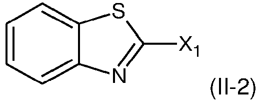 X₁ being selected from the group consisting of: SCF₃, SOCF₃, SO₂CF₃, SCF₂SO₂Ph, SOCF₂SO₂Ph, SO₂CF₂SO₂Ph, and SeCF₃.

11. The process of claim 9 or 10, wherein the reaction is carried out in the presence of toluene with bis(1,5-cyclooctadiene)nickel and Me₂(*p*-CF₃(C₆H₄))SiH.

12. The process of any one of claims 1 to 8, for the preparation of a compound having the formula (I-3): 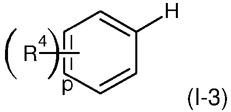 from a compound having the formula (II-3): 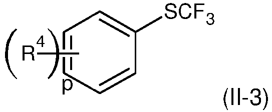
p being 0, 1, 2, or 3; and
R⁴, identical or different, being selected from the group consisting of: (C₁-C₆)alkyl, (C₁-C₆)alkoxy, and -C(=O)-O(C₁-C₆)alkyl.

13. The process of any one of claims 1 to 8, for the preparation of a compound having the formula (I-4): 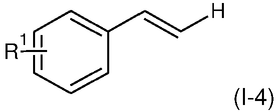 from a compound having the formula (II-4): 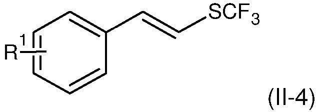 R¹ being selected from the group consisting of: halogen, (C₁-C₆)alkyl, and -O-C(=O)-(C₁-C₆)alkyl.

14. The process of claim 12 or 13, wherein the reaction is carried out in the presence of toluene, a base, and also molecular sieve, with bis(1,5-cyclooctadiene)nickel and BnMe₂SiH.

15. The process of claim 14, wherein the base is K₂HPO₄.

## Patentansprüche

1. Verfahren zur Herstellung einer Verbindung mit der Formel (I):
A-H
durch Umsetzen einer Verbindung mit der Formel (II):
A-X
in Gegenwart eines Metallkomplexes, der Nickel, einen Liganden, ein Hydrid und ein Lösungsmittel umfasst,
wobei:
- A aus der Gruppe ausgewählt ist, bestehend aus:
. (C₆-C₁₀) -Arylgruppen,
wobei die Arylgruppen optional mit mindestens einem Substituenten substituiert sind, der vorzugsweise aus der Gruppe ausgewählt ist, bestehend aus: Halogen, (C₁ -C₆)-Alkyl, (C₁-C₆) -Alkoxy, -CN, -CF₃, -OCF₃, -NRₐR_{b}>, -SiRₐR_{b}R_{c}, -BRₐR_{b}, (C₂-C₆) -Alkenyl, (C₂-C₆) -Alkinyl, -C (=O) -NRₐR_{b} und -C (=O) - O (C₁-C₆) -Alkyl;
Rₐ und R_{b} unabhängig voneinander H oder eine (C₁-C₆) - Alkylgruppe sind;
R_{c} eine (C₁-C₆) -Alkylgruppe ist;
. Heteroarylgruppen, die 5 bis 10 Atome umfassen und mindestens ein aus O, N und S ausgewähltes Heteroatom enthalten,
wobei die Heteroarylgruppen optional mit mindestens einem Substituenten substituiert sind, der vorzugsweise ausgewählt ist aus: Halogen, (C₁-C₆) -Alkyl, (C₁-C₆) -Alkoxy, - CN, -CF₃, -OCF₃, -NRₐR_{b}, -Si (R_{c}) ₃, -BRₐR_{b}, (C₂-C₆) -Alkenyl, (C₂-C₆) -Alkinyl, -C(=O) -NRₐR_{b} und -C (=O) -O (C₁-C₆) -Alkyl, wobei Rₐ, R_{b} und R_{c} wie oben definiert sind; und
. eine Vinylverbindung mit Folgendem (III): 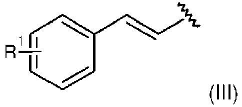 wobei R¹ aus der Gruppe ausgewählt ist, bestehend aus: Halogen, (C₁-C₆) -Alkyl, (C₁-C₆) -Alkoxy, -CN, -CF₃, -OCF₃, - NRₐR_{b}, -Si (R_{c}) ₃, -BRₐR_{b}, (C₂-C₆) -Alkenyl, (C₂-C₆) -Alkinyl, - C (=O) -NRₐR_{b} und -C (=O) -O (C₁-C₆) -Alkyl, wobei Rₐ, R_{b} und R_{c} wie oben definiert sind; und
- X aus der Gruppe ausgewählt ist, bestehend aus: YCF₃, YOCF₃, YO₂CF₃, YCF₂SO₂Ph, YOCF₂SO₂ Ph, YO₂CF₂SO₂ Ph, Y(O)ₙCF₂COOR, Y(O)ₙCF₂CONR₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR, wobei Y S oder Se ist, n 1 oder 2 ist und R (C₁-C₆)-Alkyl ist.

2. Verfahren nach Anspruch 1, wobei das Hydrid eine Silanverbindung der Formel R² (R³) ₂SiH ist, wobei R² und R³ unabhängig voneinander aus (C₁-C₆) -Alkyl-, (C₁-C₆) Alkoxy- und (C₆-C₁₀)-Arylgruppen ausgewählt sind, wobei die Arylgruppen optional mit mindestens einem Substituenten substituiert sind, der ausgewählt ist aus: (C₁-C₆) -Alkyl, (C₁-C₆) -Alkoxy, -CN, -OCF₃, -NRₐR_{b}, -Si (R_{c}) ₃, -BRₐR_{b}, (C₂-C₆) -Alkenyl, (C₂-C₆) - Alkinyl, -C (=O) -NRₐR_{b} und -C (=O) -O (C₁-C₆) -Alkyl und CF₃, wobei Rₐ, R_{b} und R_{c} wie in Anspruch 1 definiert sind.

3. Verfahren nach Anspruch 1 oder 2, wobei das Hydrid aus der Gruppe ausgewählt ist, bestehend aus: EtMe₂SiH, Me(OMe)₂SiH, Me(OEt)₂SiH, (OEt)₃SiH, Et₃SiH, tBuMe₂SiH, Me₂PhSiH, Me₂BnSiH, MePh₂SiH, Ph₃SiH, Me₂(*p*-CF₃(C₆H₄) SiH und Me₂(*p*-tBu(C₆H₄))SiH.

4. Verfahren nach einem der Ansprüche 1 bis 3, wobei das Hydrid Me₂(*p*-CF₃(C₆H₄))SiH oder Me₂BnSiH ist.

5. Verfahren nach einem der Ansprüche 1 bis 4, wobei der Ligand aus der Gruppe ausgewählt ist, bestehend aus: 1,5-Cyclooctadien (COD), 1,2-Bis(dicyclohexylphosphino)ethan (dcype), 1,1'-Bis(diphenylphosphino)ferrocen (dppf), 1,1'-Bis(di-*tert-* butylphosphino)ferrocen (DTBPF), 4,5-Bis(diphenylphosphino)-9,9-dimethylxanthen (XantPhos) und Triphenylarsin (AsPh₃).

6. Verfahren nach einem der Ansprüche 1 bis 5, wobei das Lösungsmittel Toluol ist.

7. Verfahren nach einem der Ansprüche 1 bis 6, wobei die Reaktion bei einer Temperatur von 90 °C bis 140 °C durchgeführt wird.

8. Verfahren nach einem der Ansprüche 1 bis 7, wobei der Metallkomplex ein Ni(0)-Komplex wie Bis(1,5-cyclooctadien)nickel ist.

9. Verfahren nach einem der Ansprüche 1 bis 8, zur Herstellung einer Verbindung mit der Formel (I), aus einer Verbindung mit der Formel A-SCF₃ (11-1),
wobei A eine Heteroarylgruppe ist, die 5 bis 10 Atome umfasst und mindestens ein aus O, N und S ausgewähltes Heteroatom umfasst, wobei die Heteroarylgruppe optional mit mindestens einem Substituenten substituiert ist, der ausgewählt ist aus: Halogen, (C₁-C₆) -Alkyl, (C₁-C₆) -Alkoxy und -C(=O)-O(C₁-C₆)-Alkyl.

10. Verfahren nach einem der Ansprüche 1 bis 8, zur Herstellung einer Verbindung mit der Formel (l-2): 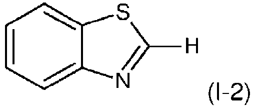
aus einer Verbindung mit der Formel (ll-2): 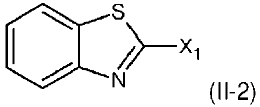
wobei X₁ aus der Gruppe ausgewählt ist, bestehend aus: SCF₃, SOCF₃, SO₂CF₃, SCF₂SO₂Ph, SOCF₂SO₂Ph, SO₂CF₂SO₂Ph und SeCF₃.

11. Verfahren nach Anspruch 9 oder 10, wobei die Reaktion in Gegenwart von Toluol mit Bis(1,5-cyclooctadien) nickel und Me₂(*p*-CF₃(C₆H₄))SiH durchgeführt wird.

12. Verfahren nach einem der Ansprüche 1 bis 8, zur Herstellung einer Verbindung mit der Formel (l-3): 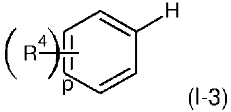
aus einer Verbindung mit der Formel (ll-3): 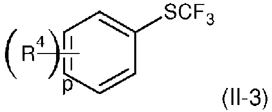
wobei p 0, 1,2 oder 3 ist; und
wobei R⁴, gleich oder verschieden, ausgewählt ist aus der Gruppe, bestehend aus: (C₁-C₆) -Alkyl, (C₁-C₆) -Alkoxy und -C(=O)-O(C₁-C₆)-Alkyl.

13. Verfahren nach einem der Ansprüche 1 bis 8, zur Herstellung einer Verbindung mit der Formel (l-4): 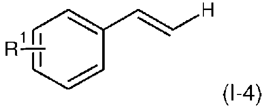
aus einer Verbindung mit der Formel (ll-4): 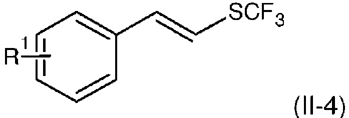
wobei R¹ aus der Gruppe ausgewählt ist, bestehend aus: Halogen, (C₁-C₆) -Alkyl und -O-C(=O)-(C₁-C₆)-Alkyl.

14. Verfahren nach Anspruch 12 oder 13, wobei die Reaktion in Gegenwart von Toluol, einer Base und auch Molekularsieb, mit Bis(1,5-cyclooctadien)nickel und BnMe₂SiH durchgeführt wird.

15. Verfahren nach Anspruch 14, wobei die Base K₂HPO₄ ist.

## Revendications

1. Procédé de préparation d'un composé de formule (I) :
A-H
par réaction d'un composé de formule (II) :
A-X
en présence d'un complexe métallique comportant du nickel, un ligand, un hydrure et un solvant,
dans lequel :
- A est sélectionné dans le groupe consistant en :
. des groupes (C₆-C₁₀)aryle,
lesdits groupes aryle étant facultativement substitués par au moins un substituant de préférence sélectionné dans le groupe consistant en : halogène, (C₁-C₆) alkyle, (C₁-C₆) alcoxy, -CN, -CF₃, -OCF₃, -NRₐR_{b}, -SiRₐR_{b}R_{c}, -BRₐR_{b}, (C₂-C₆) alcényle, (C₂-C₆) alcynyle, -C (=O) -NRₐR_{b} et -C (=O) -O (C₁-C₆) alkyle ;
Rₐ et R_{b} étant indépendamment l'un de l'autre H ou un groupe (C₁-C₆)alkyle ;
R_{c} étant un groupe (C₁-C₆)alkyle ;
. des groupes hétéroaryle comportant de 5 à 10 atomes et incluant au moins un hétéroatome sélectionné parmi O, N et S,
lesdits groupes hétéroaryle étant facultativement substitués par au moins un substituant de préférence sélectionné parmi : halogène, (C₁-C₆) alkyle, (C₁-C₆) alcoxy, - CN, -CF₃, -OCF₃, -NRₐR_{b}, -Si (R_{c}) ₃, -BRₐR_{b}, (C₂-C₆) alcényle, (C₂-C₆) alcynyle, -C (=O) -NRₐR_{b} et -C (=O) -O (C₁-C₆) alkyle, Rₐ, R_{b} et R_{c} étant tels que définis ci-dessus ; et
. un composé vinylique ayant la formule (III) suivante : 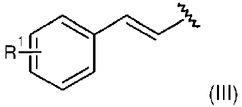 R¹ étant sélectionné dans le groupe consistant en : halogène, (C₁-C₆) alkyle, (C₁-C₆) alcoxy, -CN, -CF₃, -OCF₃, - NRₐR_{b}, -Si (R_{c}) ₃, -BRₐR_{b}, (C₂-C₆) alcényle, (C₂-C₆) alcynyle, - C (=O)-NRₐR_{b} et -C (=O) -O (C₁-C₆) alkyle, Rₐ, R_{b} et R_{c} étant tels que définis ci-dessus ; et
- X est sélectionné dans le groupe consistant en : YCF₃, YOCF₃, YO₂CF₃, YCF₂SO₂Ph, YOCF₂SO₂Ph, YO₂CF₂SO₂Ph, Y(O)ₙCF₂COOR, Y(O)ₙCF₂CONR₂, Y(O)ₙCF₂CH₂OH, Y(O)ₙCN, Y(O)ₙCHF₂, Y(O)ₙCF₂H, Y(O)ₙCF₂CₙF₂ₙ₊₁, Y(O)ₙCF₂COR, Y étant S ou Se, n étant 1 ou 2 et R étant (C₁-C₆) alkyle.

2. Procédé selon la revendication 1, dans lequel l'hydrure est un composé silane de formule R²(R³)₂SiH, R² et R³ étant sélectionnés indépendamment l'un de l'autre parmi (C₁-C₆) alkyle, (C₁-C₆) alcoxy et des groupes (C₆-C₁₀) aryle, lesdits groupes aryle étant facultativement substitués par au moins un substituant sélectionné parmi : (C₁-C₆)alkyle, (C₁-C₆)alcoxy, -CN, -OCF₃, -NRₐR_{b}, -Si(R_{c})₃, -BRₐR_{b}, (C₂-C₆) alcényle, (C₂-C₆) alcynyle, -C (=O) -NRₐR_{b} et -C (=O) -O (C₁-C₆) alkyle, et CF₃, Rₐ, R_{b} et R_{c} étant tels que définis dans la revendication 1.

3. Procédé selon la revendication 1 ou 2, dans lequel l'hydrure est sélectionné dans le groupe consistant en : EtMe₂SiH, Me(OMe) ₂SiH, Me(OEt) ₂SiH, (OEt)₃SiH, Et₃SiH, tBuMe₂SiH, Me₂PhSiH, Me₂BnSiH, MePh₂SiH, Ph₃SiH, Me₂(*p-*CF₃(C₆H₄))SiH et Me₂ (*p*-tBu(C₆H₄))SiH.

4. Procédé selon l'une quelconque des revendications 1 à 3, dans lequel l'hydrure est Me₂(*p*-CF₃(C₆H₄))SiH ou Me₂BnSiH.

5. Procédé selon l'une quelconque des revendications 1 à 4, dans lequel le ligand est sélectionné dans le groupe consistant en : 1,5-cyclooctadiène (COD), 1,2-bis(dicyclohexylphosphino)éthane (dcype), 1,1'-bis(diphénylphosphino)ferrocène (dppf), 1,1'-bis(di-*tert-*butylphosphino)ferrocène (DTBPF), 4,5-bis(diphénylphosphino)-9,9-diméthylxanthène (XantPhos) et triphénylarsine (AsPh₃).

6. Procédé selon l'une quelconque des revendications 1 à 5, dans lequel le solvant est le toluène.

7. Procédé selon l'une quelconque des revendications 1 à 6, dans lequel la réaction est réalisée à une température de 90 °C à 140 °C.

8. Procédé selon l'une quelconque des revendications 1 à 7, dans lequel le complexe métallique est un complexe Ni (0) tel que le bis(1,5-cyclooctadiène)nickel.

9. Procédé selon l'une quelconque des revendications 1 à 8, pour la préparation d'un composé de formule (I), à partir d'un composé de formule A-SCF₃ (ll-1),
dans lequel A est un groupe hétéroaryle comportant de 5 à 10 atomes et incluant au moins un hétéroatome sélectionné parmi O, N et S, ledit groupe hétéroaryle étant facultativement substitué par au moins un substituant sélectionné parmi : halogène, (C₁-C₆) alkyle, (C₁-C₆) alcoxy et -C (=O) -O (C₁-C₆) alkyle.

10. Procédé selon l'une quelconque des revendications 1 à 8, pour la préparation d'un composé de formule (l-2) : 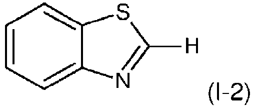
à partir d'un composé de formule (ll-2) : 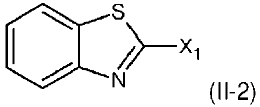
X₁ étant sélectionné dans le groupe consistant en : SCF₃, SOCF₃, SO₂CF₃, SCF₂SO₂Ph, SOCF₂SO₂Ph, SO₂CF₂SO₂Ph et SeCF₃.

11. Procédé selon la revendication 9 ou 10, dans lequel la réaction est réalisée en présence de toluène avec du bis (1, 5-cyclooctadiène) nickel et du Me₂(*p*-CF₃(C₆H₄))SiH.

12. Procédé selon l'une quelconque des revendications 1 à 8, pour la préparation d'un composé de formule (l-3) : 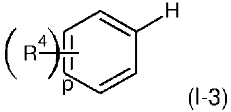
à partir d'un composé de formule (ll-3) : 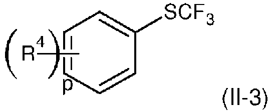
p étant 0, 1, 2 ou 3 ; et
R⁴, identique ou différent, étant sélectionné dans le groupe consistant en : (C₁-C₆) alkyle, (C₁-C₆) alcoxy et - C (=O) -O (C₁-C₆) alkyle.

13. Procédé selon l'une quelconque des revendications 1 à 8, pour la préparation d'un composé de formule (l-4) : 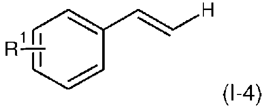
à partir d'un composé de formule (ll-4) : 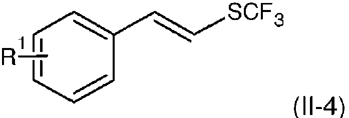
R¹ étant sélectionné dans le groupe consistant en : halogène, (C₁-C₆) alkyle et -O-C (=O) - (C₁-C₆) alkyle.

14. Procédé selon la revendication 12 ou 13, dans lequel la réaction est réalisée en présence de toluène, d'une base et également d'un tamis moléculaire, avec du bis(1,5-cyclooctadiène)nickel et du BnMe₂SiH.

15. Procédé selon la revendication 14, dans lequel la base est du K₂HPO₄.
